# EUROPEAN PATENT SPECIFICATION

(11) **EP 2 271 342 B1**
(45) Date of publication and mention of the grant of the patent: **10.10.2012**
(21) Application number: 09724965.0
(22) Date of filing: 26.03.2009
(51) Int. Cl.: A61K 31/455, A61P 17/02, A61P 17/04

(54) **USE OF NICOTINAMIDE FOR TREATING SUMMER ITCH IN HORSES**
VERWENDUNG VON NICOTINSÄUREAMID ZUR BEHANDLUNG VON SOMMEREKZEM BEI PFERDEN
UTILISATION DU NICOTINAMIDE POUR LE TRAITEMENT DE LA DERMITE ESTIVALE CHEZ LES CHEVAUX

(30) Priority: 28.03.2008 EP 08153502
(43) Date of publication of application: 12.01.2011
(73) Proprietor: Elanco Animal Health Ireland Limited, Dublin (IE)
(72) Inventor: JACKERS, Marlutje Marie Anne, B-3717 Herstappe (BE)
(74) Representative: Bassinder, Emma Marie
(86) International application number: PCT/EP2009/053572
(87) International publication number: WO 2009/118371

(56) References cited:
- WO-A-00/69426
- WO-A-98/52927
- WO-A-99/47141
- DE-A1- 19 837 928
- GB-A- 2 401 044
- US-A1- 2005 008 656
- CORREA ET AL: "Seasonal allergic dermatitis in sheep in southern Brazil caused by Culicoides insignis (Diptera: Ceratopogonidae)" VETERINARY PARASITOLOGY, ELSEVIER SCIENCE, AMSTERDAM, NL, vol. 145, no. 1-2, 13 March 2007 (2007-03-13), pages 181-185, XP005919860 ISSN: 0304-4017
- HEGYI JURAJ ET AL: "Pellagra: dermatitis, dementia, and diarrhea." INTERNATIONAL JOURNAL OF DERMATOLOGY JAN 2004, vol. 43, no. 1, January 2004 (2004-01), pages 1-5, XP002480301 ISSN: 0011-9059
- JANSSEN ANIMAL HEALTH: 'Advice on sweet itch' 2010,
- SYGALL, RICHARD: 'Managing summer skin allergies in horses' June 2010,

## Description

The present invention relates to the use of nicotinamide, as pure nicotinamide or in the form of vitamin B3, for the treatment of summer itch in horses.

Summer itch is a distressing skin condition that affects thousands of horses across the world and is also known as summer eczema, seasonal dermatitis, "Queensland itch" in Australia, "sweet itch" in Great Britain and "Kasen disease" in Japan. Summer itch is due to a hypersensitivity reaction of mammals to the contents of the saliva of gnats of the species *Culicoides,* in particular *Culicoides pulicaris.* After being bitten, some horses are allergic to a substance in the saliva of the biting midge, which causes a local reaction. This in turn causes intense irritation with severe itching, which the horse tries to relieve by rubbing and biting itself.

*Culicoides pulicaris,* which are only up to 2 mm in size, are observed in the Northern Hemisphere from March-April to October-November depending upon the weather conditions. They feed primarily at dusk and dawn, and tend to feed on the horse at specific sites, particularly around the head, tail head, withers, and base of the mane, however other areas, including the chest, back and rump can also be affected. The midges like to breed on wet land, and around rivers, lakes and standing water, so susceptible horses kept close to these conditions are more likely to be affected. Affected animals are very itchy and distressed, and rub and bite themselves intensely sometimes to the point of self-mutilation. The midge bites form blisters, which can weep, causing crusting, scabs and scaling. Prolonged rubbing and biting results in, hair loss and damage to the skin, with sometimes bleeding open sores. Occasionally secondary bacterial infections can occur. It is also not uncommon for horses to rub off their mane and upper tail hair. In the long term, skin thickening and loss of hair pigmentation may occur.

The occurrence of summer itch can be controlled by reducing the exposure of horses to the *Culicoides* midges using e.g. insect proof screens in the stable or insect repellents. Calcium soaps have been disclosed in WO-99/19491 for the treatment of inflammatory skin reactions. Anti-histamines are used but results are variable. Corticosteroids are very effective at reducing the effects of itch. Oral prednisolone can be effective when given as alternate day therapy. However with all usage of corticosteroids they can increase the risk of inducing laminitis in the horse.
US 2005/0008656 discloses compositions and methods for inhibiting insects from biting, said compositions comprise at least one vitamin and at least one herb. The list of vitamins disclosed therein includes nicotinamide. WO 98/52927 discloses a chemical composition for skin treatment comprising an active ingredient selected from nicotinamides, amide derivatives thereof, nicotinic acid, nicotinic esters and derivatives thereof. WO 99/47141 discloses skin-care compositions containing a vitamin B3 compound and their use in methods for treating and/or preventing minor skin irritations.

Nicotinamide, in addition to being known as niacinamide, is also known as 3-pyridinecarboxamide, pyridine-3-carboxamine, nicotinic acid amide, vitamin B3 and vitamin PP. It is represented by the following structural formula :

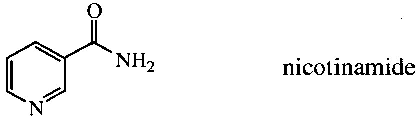

Nicotinamide is one of the two principal forms of the vitamin B3 complex which is commonly used as a collective term to refer to both nicotinamide and nicotinic acid. Nicotinamide and nicotinic acid have identical vitamin activities, but they have very different pharmacological activities.

Unexpectedly it has now been observed that nicotinamide is able to prevent, treat or cure summer itch or to reduce the symptoms of summer itch.

The present invention provides the use of nicotinamide for the manufacture of a medicament for the treatment or prevention of summer itch in horses. Nicotinamide can be used in its pure form, or in the form of vitamin B3.

Also disclosed is the use of nicotinamide for the cosmetic treatment of non-human mammals such as horses or cattle suffering from summer itch. The vitamin B3 complex is widely available in groceries, department stores and in pharmacies as a non-prescription drug since vitamin B3 is not subject to regulatory approval. Accordingly the pure cosmetic use of nicotinamide, as pure nicotinamide or in the form of vitamin B3, in the prevention or treatment of summer itch is also disclosed.

Also disclosed is a method of treating or preventing summer itch in non-human mammals which method comprises administering to said mammal a therapeutically effective amount of nicotinamide. Non-human mammals may be horses or cattle.

The term "therapeutically effective amount of nicotinamide" as used herein, means that amount of nicotinamide that elicits the biological or medicinal response in the non-human mammal that is being sought by the veterinarian, which includes alleviation of the symptoms of the condition being treated. The therapeutically effective amount can be determined using routine optimization techniques and is dependent upon the particular condition to be treated, the condition of the non-human mammal, the route of administration, the formulation, and the judgment of the practitioner and other factors evident to those skilled in the art. A therapeutically effective amount may be achieved by multiple dosing.

Those skilled in the treatment of summer itch will easily determine the therapeutically effective amount of nicotinamide from the test results presented hereinafter. In general it is contemplated that a therapeutically effective dose will be from about 0.1 mg/kg to about 10 mg/kg of body weight, more preferably from about 0.5 mg/kg to about 2 mg/kg of body weight of the horse to be treated. It may be appropriate to administer the therapeutically effective dose in the form of two or more sub-doses at appropriate intervals throughout the day.

For veterinary use nicotinamide, either as a pure nicotinamide itself or in the form of vitamin B3, can be administered as a suitably acceptable formulation in accordance with normal veterinary practice and the veterinarian will determine the dosing regimen and route of administration which will be most appropriate for a particular animal. In practice, nicotinamide for use in the treatment or prevention of summer itch is administered in the form of a solid or liquid dosage form suitable for oral or parenteral administration.

Solid dosage forms for oral or parenteral administration include capsules, dragees, tablets, powders and granules. These solid dosage forms are preferably formulated in dosage unit form for ease of administration and uniformity of dosage. "Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Liquid dosage forms for oral or parenteral administration include pharmaceutically acceptable emulsions, solutions, suspensions, suspo-emulsions, syrups and elixirs.

As an alternative the dosage forms comprising nicotinamide may be administered with the animal feedstuff and for this purpose a concentrated feed additive or premix may be prepared for mixing with the normal animal feed.

For topical application dip, spray, powder, dust, pour- on, spot-on, emulsifiable concentrate, jetting fluid, shampoos, creams, ointments, collar, tag or harness may be used. Such formulations are prepared in a conventional manner in accordance with standard veterinary and pharmaceutical practice. A drench formulation may be prepared by dispersing the active ingredients, i.e. pure nicotinamide itself or in the form of vitamin B3, in an aqueous solution together with dispersing or wetting agents and injectable formulations may be prepared in the form of a sterile solution or emulsion. Pour-on or spot-on formulations may be prepared by dissolving the active ingredients in an acceptable liquid carrier vehicle, such as butyl digol, liquid paraffin or non-volatile ester with or without addition of a volatile component such as isopropanol.

Alternatively, pour-on, spot-on or spray formulations can be prepared by encapsulation to leave a residue of active agent, i.e. pure nicotinamide itself or in the form of vitamin B3, on the surface of the animal. These formulations will vary with regard to the weight of active compound depending on the species of host animal to be treated, the severity and type of infection and type and body weight of the host.

### Experimental part

A group of five horses were treated with nicotinamide in the period from July till September. Nicotinamide was administered in the form of vitamin B3 and formulated as tablets each containing 200 mg nicotinamide. Said tablets were mixed with the horse feed for administration.

These horses, with ages ranging from 4 till 12 years, have been suffering from summer itch every year from the age as early of two. Each horse was evaluated every two weeks for symptoms of summer itch : rubbing, hair loss, damaged skin, damaged mane and root of the tail, and fresh or healed skin lesions. The seriousness of summer itch was evaluated on a scale from 1 to 10. At the start of the treatment, all five horses had summer itch symptoms ranging from 6 to 10 on said scale. After one month of treatment, the seriousness of summer itch dropped to 1 or 2 on said scale.

**Table 1 : Horses**

| Horse # | Race | Weight | Age (years) | Age when Summer itch was first seen |
|---|---|---|---|---|
| 1 | Frisian | 600 kg | 7 | 3 |
| 2 | Frisian | 550 kg | 4 | 2 |
| 3 | Frisian | 700 kg | 14 | 3 |
| 4 | Belgian Warmblood | 700 kg | 4 | 2 |
| 5 | Fjord | 450 kg | 12 | 2 |

**Table 2 : Treatment results.**

| Horse # | Dosing | Treatment period | Symptoms on summer itch scale at start | Symptoms on summer itch scale after one month |
|---|---|---|---|---|
| 1 | 300 mg/day | 7 July - 30 September | 7 | 1 |
| 2 | 300 mg/day | 7 July - 30 September | 8 | 1 |
| 3 | 600 mg/day | 21 July - 30 September | 10 | 2 |
| 4 | 500 mg/day | 21 July - 30 September | 6 | 1 |
| 5 | 500 mg/day | 21 August - 30 September | 8 | 1 |

## Claims

1. Nicotinamide for use in the treatment or prevention of skin lesions resulting from summer itch caused by *Culicoides* in horses, wherein the skin lesions are hair loss, damaged skin, damaged mane and root of the tail.

2. Nicotinamide for use according to claim 1, wherein nicotinamide is to be administered in the form of a solid dosage form suitable for oral or parenteral administration.

3. Nicotinamide for use according to claim 2, wherein the solid dosage form is a concentrated feed additive.

4. Nicotinamide for use according to claim 1, wherein nicotinamide is to be administered in the form of a liquid dosage form suitable for oral, parenteral or topical administration.

5. Nicotinamide for use according to claim 4, wherein the liquid dosage form suitable for topical administration is a cream or ointment.

6. Nicotinamide for use according to any of claims 1 to 4, wherein nicotinamide is to be administered in an amount from 0.1 mg/kg to 10 mg/kg of body weight.

7. Nicotinamide for use according to claim 6, wherein nicotinamide is to be administered in an amount from 0.5 mg/kg to 2 mg/kg of body weight.

## Patentansprüche

1. Nicotinamid zur Verwendung bei der Behandlung oder Prävention von Hautläsionen, die von einem durch *Culicoides* verursachten Sommerekzem bei Pferden herrühren, wobei die Hautläsionen Haarverlust, Schäden an Haut, Mähne und Schweifrübe umfassen.

2. Nicotinamid zur Verwendung gemäß Anspruch 1, wobei das Nicotinamid in Form einer festen Dosierungsform, die für eine orale oder parenterale Verabreichung geeignet ist, zu verabreichen ist.

3. Nicotinamid zur Verwendung gemäß Anspruch 2, wobei die feste Dosierungsform ein konzentriertes Futteradditiv ist.

4. Nicotinamid zur Verwendung gemäß Anspruch 1, wobei das Nicotinamid in Form einer flüssigen Dosierungsform, die für eine orale, parenterale oder topische Verabreichung geeignet ist, zu verabreichen ist.

5. Nicotinamid zur Verwendung gemäß Anspruch 4, wobei die zur topischen Verabreichung geeignete flüssige Dosierungsform eine Creme oder Salbe ist.

6. Nicotinamid zur Verwendung gemäß einem der Ansprüche 1 bis 4, wobei das Nicotinamid in einer Menge von 0,1 mg/kg Körpergewicht bis 10 mg/kg Körpergewicht zu verabreichen ist.

7. Nicotinamid zur Verwendung gemäß Anspruch 6, wobei das Nicotinamid in einer Menge von 0,5 mg/kg Körpergewicht bis 2 mg/kg Körpergewicht zu verabreichen ist.

## Revendications

1. Nicotinamide à utiliser dans le traitement ou dans la prévention de lésions cutanées résultant d'un eczéma d'été provoqué par *Culicoides* chez les chevaux, les lésions cutanées représentant une perte de poils, une peau endommagée, une crinière endommagée et des lésions cutanées à la base de la queue.

2. Nicotinamide à utiliser conformément à la revendication 1, dans lequel le nicotinamide doit être administré sous la forme d'une forme posologique solide appropriée pour une administration par voie orale ou par voie parentérale.

3. Nicotinamide à utiliser conformément à la revendication 2, dans lequel la forme posologique solide est un additif concentré pour l'alimentation animale.

4. Nicotinamide à utiliser conformément à la revendication 1, dans lequel le nicotinamide doit être administré sous la forme d'une forme posologique liquide appropriée pour une administration par voie orale, par voie parentérale ou par voie locale.

5. Nicotinamide à utiliser conformément à la revendication 4, dans lequel la forme posologique liquide appropriée pour une administration par voie locale est une crème ou un onguent.

6. Nicotinamide à utiliser conformément à l'une quelconque des revendications 1 à 4, dans lequel le nicotinamide doit être administré en une quantité de 0,1 mg/kg à 10 mg/kg du poids corporel.

7. Nicotinamide à utiliser conformément à la revendication 6, dans lequel le nicotinamide doit être administré en une quantité de 0,5 mg/kg à 2 mg/kg du poids corporel.
